# EUROPEAN PATENT APPLICATION

(11) **EP 4 802 990 A1**
(43) Date of publication of application: **09.09.2026**
(21) Application number: 24883768.4
(22) Date of filing: 18.03.2024
(51) Int. Cl.: A61B 3/10

(54) **INTRAOCULAR OCT OPTICAL FIBER PROBE**

(30) Priority: 03.11.2023 CN 202311454180
(71) Applicant: Beijing Xiren Technology Co., Ltd., Beijing 100123 (CN); Hangzhou Xifan Medical Device Technology Co., Ltd, Hangzhou, Zhejiang 310051 (CN)
(72) Inventor: ZHANG, Chi, Beijing 100123 (CN); CHEN, Quemin, Beijing 100123 (CN); ZHANG, Bingying, Beijing 100123 (CN)
(74) Representative: Maiwald GmbH
(86) International application number: PCT/CN2024/082308
(87) International publication number: WO 2025/091753

(57) **Abstract**

The present invention relates to an OCT-and-ophthalmic-surgical-device integration system. The system comprises an OCT probe, a display, an OCT module, a light source module and a power supply module, wherein the OCT module is used for receiving a detection signal of the OCT probe and converting the said signal into an image. The OCT probe comprises an optical fiber head end and an optical fiber handle. The optical fiber head end comprises an OCT optical fiber, an optical fiber metal sleeve and a light-guide optical fiber; the optical fiber handle comprises a rotating motor and a handle housing. A head end of the OCT optical fiber and a head end of the light-guide optical fiber are fixedly packaged inside the optical fiber metal sleeve; the optical fiber metal sleeve is further connected to the rotating motor and is driven by the rotating motor to rotate; the rotating motor is electrically connected to a power line, the power line being further electrically connected to the power supply module; at least a part of the optical fiber metal sleeve and the rotating motor are both arranged in the handle housing; a tail end of the light-guide optical fiber extends out of an end portion of the handle housing and is connected to the light source module; and a tail end of the OCT optical fiber extends out of an end portion of the handle housing and is connected to the OCT module.

## Description

### CROSS-REFERENCE TO RELATED APPLICATIONS

This application claims the benefit of priority of Chinese Patent Application No. CN 202211395260.4, entitled "Intraocular OCT Optical Fiber Probe", filed on 9 November 2022, and of Chinese Patent Application No. CN 202311454180.6, entitled "Optical Fiber Scanning Detection System and Method for Ophthalmology", filed on 3 November 2023, the entire contents of both of which are incorporated herein by reference.

### TECHNICAL FIELD

The present invention relates to the technical field of medical devices, and in particular to an intraocular OCT optical fiber probe, and to an optical fiber scanning detection system and method for ophthalmology.

### BACKGROUND

OCT (optical coherence tomography) is a medical imaging technique based on low-coherence interferometry. OCT imaging systems are currently used widely in ophthalmology and interventional cardiology. In ophthalmology, OCT systems are at present essentially outpatient examination devices, used for imaging ocular structures such as the retina before and after surgery.

Because the working principle of OCT exploits the differing reflectivities of intraocular tissues to light (typically 830 nm near-infrared light), and uses a low-coherence interferometer to compare the delay time and reflection intensity of the reflected and reference light waves, in order to analyse the structure and spacing of the different tissues, a computer is needed to process the image, which is then displayed in pseudo-colour to show the cross-sectional structure of the tissue. OCT systems therefore require a swinging mirror surface, with the result that, in the prior art, OCT systems mostly have a benchtop structure.

In other words, conventional ophthalmic OCT is a benchtop examination device that is normally housed in an examination room and is used for pre-operative quantification of intraocular lesions and for post-operative review. Application of such systems during surgery has so far taken the form of integrating the OCT into the surgical microscope so as to facilitate retinal scanning during the procedure and to assess the treatment of any intraocular lesion. Being a non-invasive examination, however, such measurement is subject to the influence of the ocular refractive media, which impairs imaging quality.

At present, invasive OCT is mainly used in interventional cardiology, where it is applied to inspection of coronary stent apposition. With respect to invasive ophthalmic examinations, the small operating space, the difficulty of providing illumination and the difficulty of controlling the scanning direction have long meant that there has been no satisfactory solution in the art.

Conventional OCT systems are already fairly well developed as far as ophthalmic examination is concerned, where they are mainly used for ophthalmic-related testing or examination. Such conventional OCT systems require a swinging mirror surface, and so the OCT sample arm tends to take the form of a lens-type scanning structure. Such a structure is influenced, in measurement, by the ocular refractive media; the measurement angle range is also affected, which in turn impacts on imaging quality. Furthermore, existing OCT systems can only change the scanning range, during a scan, by swinging the mirror surface - in other words, only by exploiting the oscillation range of a galvanometer - with the result that, in fact, in some situations the desired range or region simply cannot be scanned or detected. There is also still significant scope for improvement in several other areas, such as how the scanning detection system can be optimised, how it can be further made possible for the measurement angle range to be independent of the device, and how the influence of the ocular refractive media on the detection process can be avoided.

It is therefore necessary to provide an optical fiber scanning detection system and method for ophthalmology that can address the above problems.

### SUMMARY OF THE INVENTION

In one aspect, the present invention provides an intraocular OCT optical fiber probe; the technical problems to be solved by the invention include at least how to apply invasive OCT examination to ophthalmology, so that intraoperative OCT scanning is not influenced by the ocular refractive media, that illumination is convenient, and that the scanning direction is artificially controllable and positioning is more accurate and more convenient. At the same time, the present invention provides an optical fiber scanning detection system and method for ophthalmology, in order to solve the technical problems in the prior art that, because of the constraints imposed by the oscillation range of the galvanometer, existing OCT scanning detection systems cannot detect some of the ranges or regions that it is desired to detect, and that detection accuracy is poor because the detection process is subject to the influence of the ocular refractive media. The technical problems to be solved by the present invention are addressed by means of the following technical solutions.

To achieve the above object, a first aspect of the present invention provides an intraocular OCT optical fiber probe, comprising an OCT optical fiber, an optical fiber metal sleeve, a light-guide optical fiber, a rotating motor and a handle housing, wherein a head end of the OCT optical fiber and a head end of the light-guide optical fiber are fixedly packaged inside the optical fiber metal sleeve; the optical fiber metal sleeve is further connected to the rotating motor and is driven by the rotating motor to rotate and, via the optical fiber metal sleeve, to drive the head end of the OCT optical fiber and the head end of the light-guide optical fiber to rotate; the rotating motor is electrically connected to a power line, the power line supplying electrical power to the rotating motor; at least a part of the optical fiber metal sleeve and the rotating motor are both arranged in the handle housing; a tail end of the light-guide optical fiber extends out of an end portion of the handle housing and is connected to an illumination light source, the light-guide optical fiber being used to provide illumination; and a tail end of the OCT optical fiber extends out of an end portion of the handle housing and is connected to an OCT examination device, the OCT optical fiber being used to perform intraocular invasive OCT examination.

According to an optional embodiment, a gap between the head end of the OCT optical fiber and the head end of the light-guide optical fiber and an inner wall of the optical fiber metal sleeve is filled with AB glue (i.e., a two-component adhesive).

According to an optional embodiment, the rotating motor drives the optical fiber metal sleeve to rotate, rather than rotating the optical fiber.

According to an optional embodiment, the light-guide optical fiber is a multimode optical fiber.

According to an optional embodiment, the light-guide optical fiber is a single-mode optical fiber.

According to an optional embodiment, rotation of the optical fiber metal sleeve causes a scanning rotation angle of the intraocular OCT optical fiber to be from 1° to 360°.

According to an optional embodiment, the OCT optical fiber comprises a single-mode optical fiber, a spring tube, a glass rod, a self-focusing lens and a reflector, the single-mode optical fiber being received in the spring tube; one end of the glass rod is cemented to the self-focusing lens at zero degrees, and the other end of the glass rod is obliquely cemented to the single-mode optical fiber, so that the working distance of the OCT probe can be changed by altering the cementing distance between the glass rod and the single-mode optical fiber to achieve a desired working distance, thereby increasing the numerical aperture and lateral resolution of the OCT probe; the single-mode optical fiber, the spring tube, the glass rod, the self-focusing lens and the reflector are sealed within the optical fiber metal sleeve, the reflector being used to reduce the influence on imaging of stray light from the light source that passes through a cylindrical inner tube of the optical fiber metal sleeve.

According to an optional embodiment, the optical fiber metal sleeve is a slotted stainless steel tube, a slot being provided in a side wall of the optical fiber metal sleeve, and a reflective surface of the reflector being directed toward an opening of the slot to reduce the influence on imaging of stray light from the light source that passes through the cylindrical inner tube of the optical fiber metal sleeve.

According to an optional embodiment, the reflector is a cylindrical reflector.

According to an optional embodiment, an end portion of the optical fiber metal sleeve is sealed with UV glue.

Compared with the prior art, the advantageous effects of the present invention are as follows:
The OCT optical fiber probe of the present invention applies OCT to intraocular examination, can be integrated with the light-guide optical fiber and is able to carry out OCT examination while using conventional illumination, so that intraoperative OCT scanning is not influenced by the ocular refractive media.

The intraocular OCT optical fiber of the present invention is able to change the working distance of the OCT probe by altering the cementing distance between the glass rod 3 and the single-mode optical fiber 1, in order to achieve a desired working distance, thereby increasing the numerical aperture and lateral resolution of the OCT probe; by causing the reflective surface of the reflector 5 to face the opening of the slotted stainless steel tube 6, it is possible to reduce the influence on imaging of stray light from the light source that passes through the cylindrical inner tube.

A second aspect of the present invention provides an optical fiber scanning detection system for ophthalmology, comprising: a detection device for automatically detecting an object to be examined and a positioning point thereof; an OCT scanning optical fiber assembly, one end portion of which is connected to a lens structure, the OCT scanning optical fiber assembly being used to scan the position of the object to be examined and the position of the positioning point; a manipulator, used to grip and carry the OCT scanning optical fiber assembly in order to scan the object to be examined and further to set down the OCT scanning optical fiber assembly at the positioning point or an indicated point of the object to be examined; a controller, electrically connected to the detection device and the manipulator, the controller being used to control the detection device to perform automatic detection of the object to be examined and the positioning point thereof, and the controller being used to control the manipulator to set down the OCT scanning optical fiber assembly at the positioning point or the indicated point of the object to be examined; and wherein the OCT scanning optical fiber assembly forms an implant-type detection structure with the object to be examined.

According to an optional embodiment, the lens structure includes a first lens, the optical axis direction of the first lens making a specified angle with the cross section of the OCT scanning optical fiber assembly, the specified angle being in the range of 0° to 90°.

According to an optional embodiment, the said one end portion of the OCT scanning optical fiber assembly is connected to the first lens, so that the said specified angle between the optical axis direction of the first lens and the cross section of the OCT scanning optical fiber assembly is from 20° to 90°.

According to an optional embodiment, the OCT scanning optical fiber assembly includes a detection engagement portion; the controller, on the basis of a region to be detected of the object to be examined, controls the manipulator to set down the detection engagement portion at the positioning point or the indicated point of the object to be examined.

According to an optional embodiment, the detection engagement portion of the OCT scanning optical fiber assembly and the positioning point or indicated point of the object to be examined form an implant-type detection structure, the implant-type detection structure including a detection angle formed between the detection engagement portion and the region to be detected of the object to be examined.

According to an optional embodiment, the controller is further used to determine a direction of movement of the detection engagement portion of the OCT scanning optical fiber assembly on the basis of the automatically detected object to be examined and the positioning point thereof, and the region to be detected of the object to be examined.

According to an optional embodiment, the controller is used to control the OCT scanning optical fiber assembly to move rectilinearly along the determined direction so as to adjust the detection angle, formed between the detection engagement portion and the region to be detected of the object to be examined, to lie within a set range; alternatively, the controller is used to control the OCT scanning optical fiber assembly to move rectilinearly along the determined direction and then to move circumferentially with respect to a centre of the object to be examined, so as to adjust the detection angle, formed between the detection engagement portion and the region to be detected of the object to be examined, to lie within a set range.

According to an optional embodiment, the object to be examined is a sphere; the optical fiber scanning system further comprises an illumination optical fiber, the illumination optical fiber being used to convey light from a visible light source for illuminating the region to be detected of the object to be examined.

A third aspect of the present invention provides an optical fiber scanning imaging method for ophthalmology, performed by using the optical fiber scanning detection system according to the first aspect of the present invention, the optical fiber scanning imaging method comprising: using the detection device to detect automatically the object to be examined and the positioning point thereof; determining, on the basis of the region to be detected and the determined positioning point, a direction of movement of the detection engagement portion of the OCT scanning optical fiber assembly relative to the said region to be detected, the said direction of movement including a rectilinear direction and/or a circumferential direction determined by the positioning point and a first boundary point and a second boundary point of the region to be detected; and controlling, by means of the controller, the manipulator to grip and carry the OCT scanning optical fiber assembly so as to move along the determined rectilinear direction and/or circumferential direction and to perform scanning detection on the object to be examined, in order to obtain a detection image of the region to be detected of the object to be examined.

Embodiments of the present invention include the following advantages:
Compared with the prior art, the present invention automatically detects the object to be examined and the positioning point thereof by means of the detection device; by means of the controller, the manipulator is controlled to grip and carry the OCT scanning optical fiber assembly so as to scan the object to be examined, and further the OCT optical fiber is set down at the positioning point or indicated point of the object to be examined, forming an implant-type detection structure with the object to be examined, in order to perform scanning detection on the region to be detected of the object to be examined; in this way it is possible to improve detection accuracy of the region to be detected, and to enlarge the scanning detection range.

### BRIEF DESCRIPTION OF THE DRAWINGS

The drawings serve to provide a further understanding of the technical solutions of the present invention, and form a part of the description; they are used, together with specific embodiments of the application, to explain the technical solutions of the present invention, and are not intended to limit the technical solutions of the present invention.
- **FIG. 1**: is a schematic view of the internal structure of the intraocular OCT optical fiber according to the present invention;
- **FIG. 2**: is a schematic view of the overall structure of the intraocular OCT optical fiber according to the present invention;
- **FIG. 3**: is a structural schematic view of one embodiment of the intraocular OCT optical fiber;
- **FIG. 4**: is a structural schematic view of another embodiment of the intraocular OCT optical fiber;
- **FIG. 5**: is a schematic view of one example of the optical fiber scanning detection system for ophthalmology according to the present invention;
- **FIG. 6**: is a schematic view of an application scenario of the optical fiber scanning detection system for ophthalmology according to the present invention;
- **FIG. 7**: is a structural schematic view of an applied example of the optical fiber scanning detection system for ophthalmology according to the present invention;
- **FIG. 8**: is a partial structural schematic view of the OCT scanning optical fiber and a scanning needle of the OCT scanning optical fiber assembly in the optical fiber scanning detection system for ophthalmology according to the present invention;
- **FIG. 9**: is a partial structural enlarged schematic view of one end portion of the OCT scanning optical fiber assembly in the optical fiber scanning detection system for ophthalmology according to the present invention;
- **FIG. 10**: is a structural schematic view of a specific embodiment of FIG. 7;
- **FIG. 11**: is a structural schematic view of another specific embodiment of FIG. 7;
- **FIG. 12**: is a flow schematic view of one example of the optical fiber scanning imaging method for ophthalmology according to the present invention.

### DETAILED DESCRIPTION OF THE EMBODIMENTS

The present invention is described in greater detail below in order to facilitate understanding of the invention.

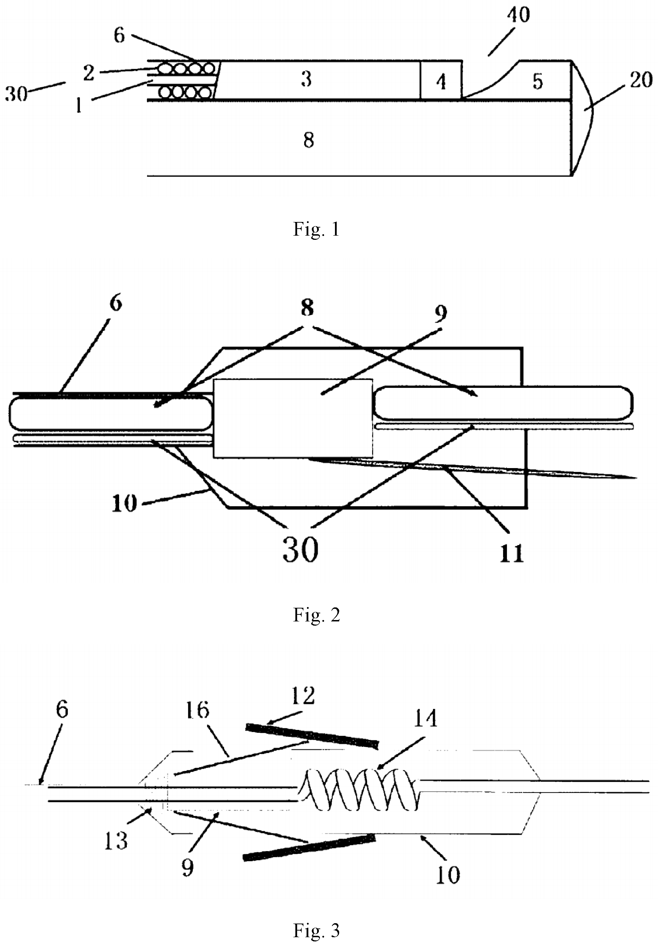

As shown in FIG. 1 and FIG. 2, the intraocular OCT optical fiber according to the present invention comprises an OCT optical fiber 30, an optical fiber metal sleeve 6, a light-guide optical fiber 8, a rotating motor 9 and a handle housing 10. A head end of the OCT optical fiber 30 and a head end of the light-guide optical fiber 8 are fixedly packaged inside the optical fiber metal sleeve 6. The optical fiber metal sleeve 6 is further connected to the rotating motor 9 and is driven by the rotating motor 9 to rotate and, via the optical fiber metal sleeve 6, to drive the head end of the OCT optical fiber 30 and the head end of the light-guide optical fiber 8 to rotate. The rotating motor 9 is electrically connected to a power line 11, the power line 11 supplying electrical power to the rotating motor 9. At least a part of the optical fiber metal sleeve 6 and the rotating motor 9 are both arranged in the handle housing 10. A tail end of the light-guide optical fiber 8 extends out of an end portion of the handle housing 10 and is connected to an illumination light source, the light-guide optical fiber 8 being used to provide illumination. A tail end of the OCT optical fiber 30 extends out of an end portion of the handle housing 10 and is connected to an OCT examination device, the OCT optical fiber 30 being used to carry out intraocular invasive OCT examination.

Preferably, a gap between the head end of the OCT optical fiber 30 and the head end of the light-guide optical fiber 8 and an inner wall of the optical fiber metal sleeve 6 is filled with AB glue (i.e., a two-component adhesive).

In the present application, the rotating motor 9 drives the optical fiber metal sleeve 6 to rotate, rather than rotating the optical fiber. The advantage of such a configuration is that rotating the optical fiber metal sleeve renders the entire intraocular OCT optical fiber more stable, thereby ensuring that the OCT examination is more accurate. By contrast, directly rotating the optical fiber would lead to relatively pronounced wobble of the optical fiber and would impair the accuracy of the OCT examination.

In one preferred embodiment, the light-guide optical fiber 8 is a multimode optical fiber.

In another preferred embodiment, the light-guide optical fiber 8 is a single-mode optical fiber.

Preferably, rotation of the optical fiber metal sleeve 6 causes a scanning rotation angle of the intraocular OCT optical fiber to be from 1° to 360°.

The schematic view shown in FIG. 2 is given purely for illustrative purposes. A person of ordinary skill in the art will fully understand that the head end and the tail end of the OCT optical fiber 30 are joined to one another to form an integral optical fiber, and the head end and the tail end of the light-guide optical fiber 8 are likewise joined to one another to form an integral optical fiber. A person of ordinary skill in the art will understand that no connecting relationship between the OCT optical fiber 30 and the light-guide optical fiber 8, on the one hand, and the rotating motor 9, on the other hand, is required: it is sufficient to provide a clearance groove in a housing of the rotating motor 9 to allow the OCT optical fiber 30 and the light-guide optical fiber 8 to pass through, and no technical difficulty needs to be overcome to do so.

Preferably, the OCT optical fiber 30 comprises a single-mode optical fiber 1, a spring tube 2, a glass rod 3, a self-focusing lens 4 and a reflector 5, the single-mode optical fiber 1 being received in the spring tube 2. One end of the glass rod 3 is cemented to the self-focusing lens 4 at zero degrees, and the other end of the glass rod 3 is obliquely cemented to the single-mode optical fiber 1, so that the working distance of the OCT probe can be changed by altering the cementing distance between the glass rod 3 and the single-mode optical fiber 1 to achieve a desired working distance, thereby increasing the numerical aperture and lateral resolution of the OCT probe. The single-mode optical fiber 1, the spring tube 2, the glass rod 3, the self-focusing lens 4 and the reflector 5 are sealed within the optical fiber metal sleeve 6, the reflector 5 being used to reduce the influence on imaging of stray light from the light source that passes through the cylindrical inner tube of the optical fiber metal sleeve 6.

Preferably, the optical fiber metal sleeve 6 is a slotted stainless steel tube; a slot 40 is provided in a side wall of the optical fiber metal sleeve 6, and the reflective surface of the reflector 5 is directed toward the opening of the slot 40, in order to reduce the influence on imaging of stray light from the light source that passes through the cylindrical inner tube of the optical fiber metal sleeve 6.

Preferably, the reflector 5 is a cylindrical reflector, and at least one reflective surface of the reflector makes a non-90° angle with the optical fiber.

Preferably, the end portion of the optical fiber metal sleeve 6 is sealed with UV glue 20.

The spring tube 2 is used to protect the single-mode optical fiber 1.

The intraocular OCT optical fiber according to the present invention makes it possible to apply the OCT optical fiber to ophthalmology, with intraoperative OCT scanning not being influenced by the ocular refractive media, with the scanning direction being artificially controllable, with positioning that is more accurate and more convenient, and with confirmation of the surgical objective being achievable intraoperatively, obviating the need for post-operative confirmation.

In the present invention, the rotating motor drives the optical fiber metal sleeve 6 to rotate, rather than rotating the optical fiber, and the scanning rotation angle is from 1° to 360°.

Because, in the prior art, the OCT is integrated within the microscope, during an ophthalmic OCT examination the OCT optical fiber does not directly contact the eye (the interior of the eye includes structures such as the cornea, the lens, the aqueous humour, etc.); the cornea must first be scanned and the aqueous humour scanned afterwards. By contrast, in the present application the OCT probe is inserted directly into the eye to scan the retina, and is therefore not influenced by the ocular refractive media.

In the embodiment shown in FIG. 3, the intraocular OCT optical fiber according to the present invention further comprises a sliding bar 12 and a connecting rod 16. One end of the sliding bar 12 is fixedly arranged on an outer peripheral wall of the handle housing 10, and the other end of the sliding bar 12 extends obliquely upward in a direction away from the handle housing 10. One end of the connecting rod 16 is connected to the sliding bar 12, and the other end of the connecting rod 16 is connected to a fiber fixing shaft. By depressing the sliding bar 12, the connecting rod 16 drives the fiber fixing shaft back and forth, thereby adjusting the length of an optical fiber 14.

In one preferred embodiment, the fiber fixing shaft is the rotating motor 9.

A spring 13 is also provided at one end portion of the rotating motor 9 close to the optical fiber metal sleeve 6.

A helical structure is further provided on a portion of the optical fiber 14 that lies on one side of the fiber fixing shaft.

The helical structures number zero or more.

The optical fiber 14 comprises the OCT optical fiber 30 and the light-guide optical fiber8.

By depressing the sliding bar 12, it is possible, via the connecting rod 16, to drive the rotating motor 9 back and forth; the rotating motor 9 is then able to drive the optical fiber 14 and the optical fiber metal sleeve 6 back and forth, thereby rendering the OCT optical fiber retractable and facilitating adjustment of the working distance of the OCT optical fiber within the eye by the physician.

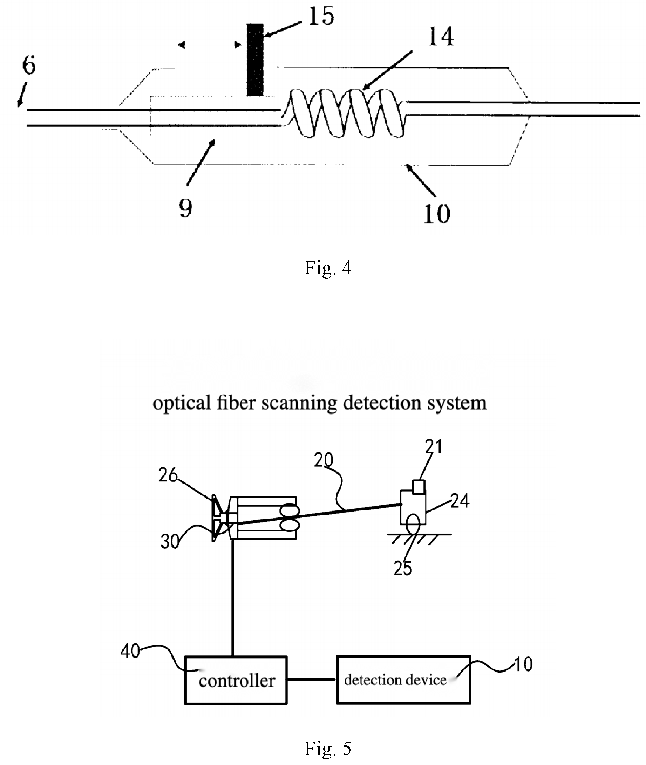

In the embodiment shown in FIG. 4, the intraocular OCT optical fiber according to the present invention further comprises a push rod 15; a sliding groove for back-and-forth movement of the push rod 15 is provided on the outer peripheral wall of the handle housing 10, the push rod 15 being received in the sliding groove and an inner end portion of the push rod 15, lying inside the handle housing 10, being connected to the rotating motor 9.

By moving the push rod 15 back and forth in the direction of the double-headed arrow in FIG. 4, it is possible to drive the rotating motor 9 back and forth, and the rotating motor 9 is able to drive the optical fiber 14 and the optical fiber metal sleeve 6 back and forth, thereby rendering the OCT optical fiber retractable and facilitating adjustment of the working distance of the OCT optical fiber within the eye by the physician.

A helical structure is further provided on a portion of the optical fiber 14 that lies on one side of the fiber fixing shaft.

The helical structures number zero or more.

The optical fiber 14 comprises the OCT optical fiber 30 and the light-guide optical fiber 8.

Key technical features of the present application include at least the following:
1. The present application applies the OCT optical fiber to ophthalmology.
2. In the present application, the rotating motor drives the optical fiber metal sleeve to rotate, rather than rotating the optical fiber.
3. In the present application, the scanning rotation angle is from 1° to 360°.
4. In the present application, the OCT optical fiber and the light-guide optical fiber are combined, addressing the problem of illumination.
5. The present application makes the OCT optical fiber probe retractable.

Referring to FIG. 5, FIG. 6, FIG. 7, FIG. 8, FIG. 9, FIG. 10 and FIG. 11, the first aspect of the present invention provides a schematic view of an example of an optical fiber scanning detection system for ophthalmology.

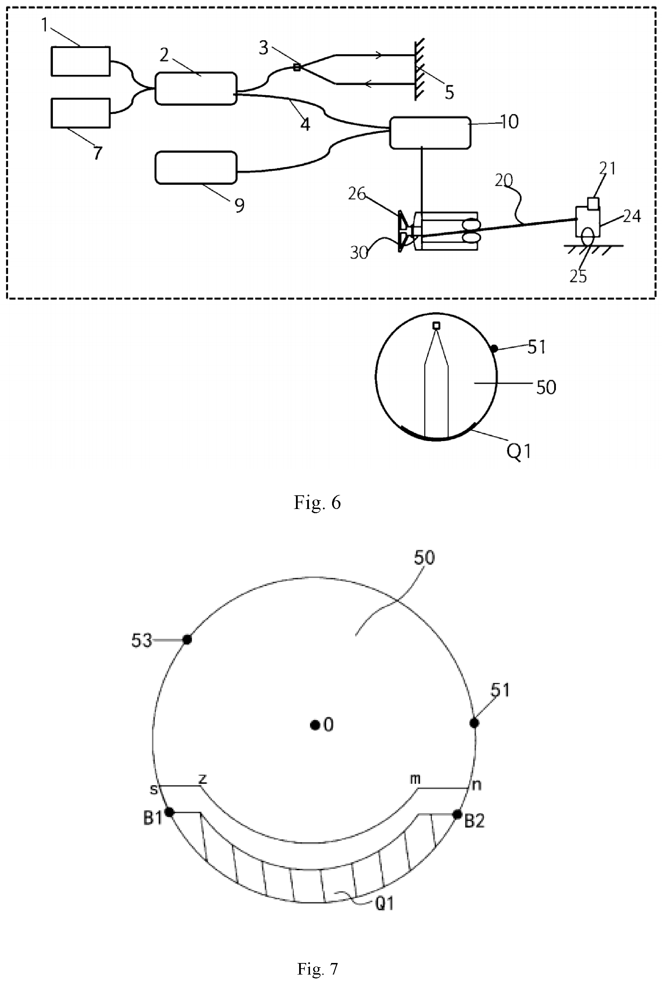

FIG. 5 is a schematic view of one example of the optical fiber scanning detection system for ophthalmology of the present invention. FIG. 6 is a schematic view of an application scenario of the optical fiber scanning detection system for ophthalmology of the present invention.

In the application scenario of FIG. 6, the optical fiber scanning detection system is used for ophthalmic examinations, in particular comprising fundus examination, local-region examination of the eyeball, fundus scanning imaging, and so on. Specifically, in the example of FIG. 6, the controller controls the manipulator to grip and carry the OCT scanning optical fiber assembly to move along the determined rectilinear direction and/or circumferential direction, and to perform scanning detection on the object to be examined (for example, a spherical object to be examined), in order to obtain a detection image of the region to be detected of the object to be examined.

It is to be noted that the above is provided only by way of an optional illustrative example, and is not to be construed as limiting the present invention.

As shown in FIG. 5, the optical fiber scanning detection system of the present invention comprises a detection device 10, an OCT scanning optical fiber assembly 20, a manipulator 30 and a controller 40, wherein one end portion of the OCT scanning optical fiber assembly 20 is connected to a lens structure, and the said one end portion of the OCT scanning optical fiber assembly 20 is further provided with a detection engagement portion 21.

Specifically, the detection engagement portion 21 is placed at the positioning point 51 of the object to be examined 50 as shown in FIG. 6 (which in this illustration also includes an indicated point), so that the detection engagement portion 21 of the OCT scanning optical fiber assembly 20 moves along a determined direction of movement y1 and along a movement path L1 in order to complete detection of the region to be detected.

More specifically, the object to be examined 50 is a spherical tissue, for example an eyeball. The positioning point 51 of the object to be examined 50 is, for example, a point determined in accordance with the user's needs, or a point determined on the basis of the region to be detected, or a point selected by the user.

In one specific embodiment, the OCT scanning optical fiber assembly 20 is used to scan the position of the object to be examined 50 and the position of the positioning point 51. The detection device is used for automatically detecting the object to be examined 50 and the positioning point 51 thereof. The said detection device may, for example, be an infrared light source, a position sensor, or the like.

Specifically, one end portion of the OCT scanning optical fiber assembly 20 is connected to a lens structure. The OCT scanning optical fiber assembly 20 includes a first end portion 24 and a second end portion 26 opposite to the first end portion 24. The lens structure includes a first lens 25, the optical axis direction G of the first lens 25 making a specified angle a with the cross section of the OCT scanning optical fiber assembly 20, the specified angle a being in the range of 0° to 90°.

Optionally, the said one end portion (specifically, the first end portion 24) of the OCT scanning optical fiber assembly 20 is connected to the first lens 25, so that the said specified angle a, formed between the optical axis direction of the first lens 25 and the cross section of the OCT scanning optical fiber assembly 20, is from 20° to 90°. Preferably, the said specified angle a is 45°.

In this embodiment, the optical fiber scanning detection system further comprises a manipulator 30, the manipulator being used to grip and carry the OCT scanning optical fiber assembly 20 to perform scanning of the object to be examined 50, and further to set down the OCT scanning optical fiber assembly 20 (specifically, the detection engagement portion 21) at the positioning point 51 or indicated point 53 of the object to be examined 50. The manipulator 30 includes a gripping portion and a locking portion. When the gripping portion of the manipulator 30 has gripped the detection engagement portion 21 of the OCT scanning optical fiber assembly 20, the detection engagement portion 21 is automatically locked via the locking portion.

To optimise the automation and intelligent operation of the optical fiber scanning detection system, the optical fiber scanning detection system further comprises a controller 40, which is electrically connected to the detection device 10 and the manipulator 30. The controller 40 is used for controlling the detection device 10 to perform automatic detection of the object to be examined 50 and the positioning point 51 thereof, and the controller 40 is used for controlling the manipulator 30 to set down the OCT scanning optical fiber assembly 20 at the positioning point 51 or the indicated point 53 of the object to be examined 50, so that the OCT scanning optical fiber assembly 20 forms an implant-type detection structure with the object to be examined 50, in order to perform detection of the region to be detected of the object to be examined 50.

FIG. 7 illustrates a local side schematic view, from one angle, of an example of the application of the optical fiber scanning detection system of the present invention.

As shown in FIG. 7, the controller 40, on the basis of the region to be detected (for example, a local region Q1 at the bottom of the eyeball, for example the diagonal-shaded region shown in FIG. 7) of the object to be examined 50, controls the manipulator 30 to set down the detection engagement portion 21 at the positioning point 51 and the positioning point 53 of the object to be examined 50 (which in the example of FIG. 7 are two positioning points). The region to be detected (for example, the local region Q1 at the bottom of the eyeball) is a curved-surface region, the region to be detected including a first boundary point B1 and a second boundary point B2.

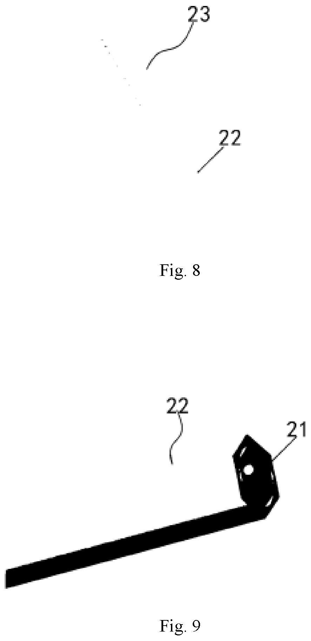

FIG. 8 illustrates a partial schematic view of the OCT scanning optical fiber and the scanning needle of the OCT scanning optical fiber assembly in the optical fiber scanning detection system of the present invention.

As shown in FIG. 8, in this embodiment, the OCT scanning optical fiber assembly 20 includes an OCT scanning optical fiber 22 and a scanning needle 23. The OCT scanning optical fiber 22 is enclosed within the scanning needle 23, and one end of the OCT scanning optical fiber 22 is exposed outside the scanning needle 23.

Specifically, the material of the scanning needle 23 may, for example, be a 304 stainless steel tube, a 316 stainless steel tube, a 420 stainless steel tube, a 17-4PH stainless steel tube, or any of various other stainless steel tubes; a nickel-titanium alloy, a cobalt-chromium alloy, a titanium alloy, or any of various other alloys; or the like.

In one specific embodiment, one end portion of the scanning needle 23 is engaged with and connected to the detection engagement portion 21, so that the detection engagement portion 21 is set down at the positioning point 51 or the indicated point 53 of the object to be examined 50.

Specifically, the one end portion of the scanning needle 23 and the detection engagement portion 21 may together perform circumferential movement with a specified radius, in order to form a scanning path along the line szmn shown in FIG. 7.

Optionally, the one end portion of the scanning needle 23 and the detection engagement portion 21 may together perform rectilinear movement in a certain direction.

FIG. 9 is a partial structural enlarged schematic view of one end portion of the OCT scanning optical fiber assembly in the optical fiber scanning detection system for ophthalmology of the present invention.

As shown in FIG. 9, in this example, one end portion of the OCT scanning optical fiber assembly 20 has the detection engagement portion 21; the detection engagement portion 21 is, for example, a flexible engagement portion connected to the first lens. The detection engagement portion 21 is arranged on the outside of the scanning needle, or may be enclosed within the scanning needle; the function of the detection engagement portion 21 is to set the OCT scanning optical fiber assembly down at the region to be detected and to assist the first lens in better returning the light reflected from the region to be detected to the OCT scanning optical fiber assembly. The front end of the OCT scanning optical fiber assembly 20 (i.e., the end corresponding to the first end portion) may rotate freely through 360°.

It is to be noted that the above is provided only by way of an optional illustrative example, and is not to be construed as limiting the present invention. In other embodiments, the detection engagement portion 21 may further include other portions for scanning, detection and the like.

Furthermore, the controller 40 is further used for determining the direction of movement of the detection engagement portion 21 of the OCT scanning optical fiber assembly 20 on the basis of the automatically detected object to be examined 50 and the positioning point 51 thereof, and the region to be detected of the object to be examined 50. The positioning point is determined on the basis of the region to be detected Q1 of the object to be examined 50, or an indicated point is selected by the user. Specifically, the positioning point is determined on the basis of the first boundary point B1 and the second boundary point B2 of the region to be detected Q1, the said positioning point being one or two in number.

Optionally, when the area of the region to be detected is less than a specified value, the number of positioning points of the object to be examined is one. When the area of the region to be detected is greater than or equal to the specified value, the number of positioning points of the object to be examined is two. The said specified value lies, for example, in the range of from 0 to 10 mm. For example, the said specified value is 2 mm in the case where the region to be detected is a problem region such as the macular region.

In the example of FIG. 7, the position of the positioning point 51, lying on the right-hand side, is further away from the centre O of the object to be examined 50 than the second boundary point B2 of the region to be detected Q1. The position of the indicated point 53, lying on the left-hand side, is closer to the centre O of the object to be examined 50 than the first boundary point B1 of the region to be detected Q1.

The detection step Δz is expressed by the following formula: $Δz = \left(2 ln 2⋅{{λ}_{0}}^{2}\right) / \left(π⋅Δλ\right)$ where Δz denotes the scanning step in the horizontal direction of the OCT scanning optical fiber in the optical fiber scanning detection system of the present invention, i.e., the detection step, in micrometres; λ₀ denotes the central wavelength of the OCT scanning light source, in nanometres; Δλ denotes the central spectral bandwidth of the OCT scanning light source, in nanometres; and π denotes the circular constant.

It is to be noted that in other examples, the position of the positioning point 51, lying on the right-hand side, is vertically aligned with the second boundary point B2 of the region to be detected Q1. The position of the indicated point, lying on the left-hand side, is vertically aligned with the first boundary point B1 of the region to be detected Q1. The above is provided only by way of illustration and is not to be construed as limiting the present invention.

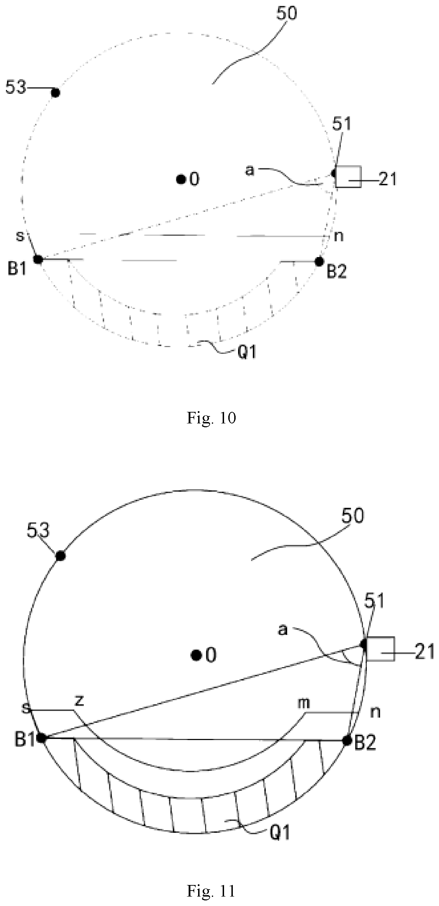

Specifically, the detection engagement portion 21 of the OCT scanning optical fiber assembly 20 and the positioning point 51 or indicated point 53 of the object to be examined 50 form an implant-type detection structure, the implant-type detection structure including the detection angle formed between the detection engagement portion 21 and the region to be detected Q1 of the object to be examined 50, as shown by the detection angle a in FIG. 10.

Specifically, the detection angle a is formed by the mutually connecting positioning point 51, first boundary point B1 of the region to be detected Q1 and second boundary point B2, i.e., the detection angle a is formed by the line joining positioning point 51 to the first boundary point B1 and the line joining positioning point 51 to the second boundary point B2; the detection angle a is in the range of from 20° to 85°, preferably from 30° to 75°.

In one specific embodiment, the controller 40 is used to control the OCT scanning optical fiber assembly 20 to move rectilinearly along a determined direction (for example, along a direction parallel to the line segment sn of FIG. 6) (specifically, to move rectilinearly along the line segment sn), so as to adjust the detection angle a, formed between the detection engagement portion 21 and the region to be detected Q1 of the object to be examined 50, to lie within the set range, and to complete the detection process for the region to be detected Q1.

In another embodiment, the controller 40 is used to control the OCT scanning optical fiber to move rectilinearly along a determined direction (for example, along a direction parallel to the line segment sz of FIG. 7) and then to move circumferentially with respect to the centre O of the object to be examined (specifically, along a curve zm), so as to adjust the detection angle a, formed between the detection engagement portion 21 and the region to be detected Q1 of the object to be examined, to lie within the set range.

In a further specific embodiment, the optical fiber scanning detection system comprises a detection device comprising an OCT infrared light source 1 and a detector 7, which is connected via a first optical fiber coupler 2 (carrying out splitting, specifically dividing the infrared light source into two beams, each beam having the same light intensity, namely 50% of the infrared light source power) to a reference arm 3, the two beams respectively passing via the reference arm 3 and a sample arm 4 to a plane mirror and the positioning point of the object to be examined; specifically, 50% of the infrared light beam is reflected via the reference arm 3, by way of the plane mirror 5, while the 50% of the infrared light beam that passes via the sample arm 4 (for example, the OCT optical fiber) carries out scanning detection of the object to be examined (for example, the eyeball). The light beam from the reference arm 3 returns by the same path, via the plane mirror 5, to the first optical fiber coupler 2, and the first optical fiber coupler 2 conveys the light beam to the detector 7. The light beam from the sample arm 4 is scanned onto the object to be examined, and the light beam reflected from the object to be examined then returns, by the same path, to the first optical fiber coupler 2, which conveys it to the detector 7. In addition, a visible light source 9 is also included. Specific reference may be made to FIG. 6.

It is to be noted that with regard to the splitting carried out via the first optical fiber coupler 2, it is also possible to divide the infrared light source into two beams having different proportions, for example a ratio of from 1:10 to 9:10. The above is provided only by way of optional illustration and is not to be construed as limiting the present invention.

In this embodiment, the optical fiber scanning system further comprises an illumination optical fiber, the illumination optical fiber being used to convey light from the visible light source for illuminating the region to be detected of the object to be examined.

For example, the visible light source 9 passes via the illumination optical fiber and, by way of a second optical fiber coupler 10, reaches the bottom of the object to be examined, for example reaches the bottom of the eyeball; this illuminates the fundus, and makes it convenient to observe the surface of the fundus under the microscope. The visible light source 9 and the scanning optical fiber of the sample arm 4 may be coupled via the second optical fiber coupler 10, or may be two separate optical fibers fixedly enclosed together by a metal sleeve.

It is to be noted that the above is provided only by way of optional illustration, and is not to be construed as limiting the present invention.

Compared with the prior art, the present invention automatically detects the object to be examined and the positioning point thereof by means of the detection device; by means of the controller, the manipulator is controlled to grip and carry the OCT scanning optical fiber assembly to perform scanning of the object to be examined, and the OCT scanning optical fiber assembly is further set down at the positioning point or indicated point of the object to be examined, forming an implant-type detection structure with the object to be examined, in order to perform scanning detection of the region to be detected of the object to be examined; in this way it is possible to improve detection accuracy of the region to be detected, to solve the problem of inability to detect with precision certain regions to be detected due to the constraints of the oscillation range of the galvanometer, and to enlarge the scanning detection range.

Below is a description of a method embodiment of the present invention; the optical fiber scanning detection system of the first aspect of the present invention is particularly suitable for the optical fiber scanning imaging method of the present invention. As regards details not disclosed in the system embodiments of the present invention, reference is made to the system embodiments of the present invention.

Referring to FIG. 5, FIG. 6, FIG. 7, FIG. 10, FIG. 11 and FIG. 12, the optical fiber scanning imaging method of the present invention is performed by using the optical fiber scanning detection system of the present invention.

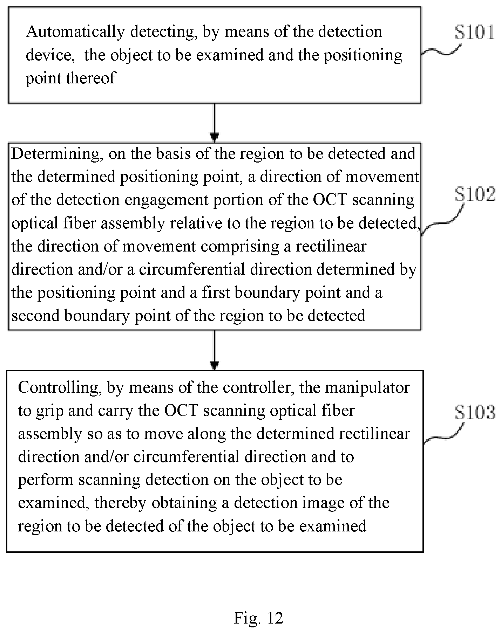

As shown in FIG. 12, the optical fiber scanning imaging method comprises the following steps:
Step S101: using the detection device automatically to detect the object to be examined and the positioning point thereof.

For example, a detection device such as an OCT infrared light source or a position sensor is used automatically to detect the object to be examined and the positioning point thereof, the object to be examined being, for example, an eyeball.

In one optional embodiment, by means of the controller, the manipulator is controlled to set down the OCT scanning optical fiber assembly at the positioning point or indicated point of the object to be examined, so that the OCT scanning optical fiber assembly forms an implant-type detection structure with the object to be examined.

Specifically, the detection engagement portion of the OCT scanning optical fiber assembly forms an implant-type detection structure with the positioning point or indicated point of the object to be examined, the implant-type detection structure including the detection angle formed between the detection engagement portion and the region to be detected of the object to be examined.

Step S102: determining, on the basis of the region to be detected and the determined positioning point, the direction of movement of the detection engagement portion of the OCT scanning optical fiber assembly with respect to the said region to be detected, the said direction of movement comprising a rectilinear direction and/or a circumferential direction determined by the positioning point and a first boundary point and a second boundary point of the region to be detected.

As can be seen from FIG. 6, the controller, on the basis of the region to be detected Q1 and the determined positioning point 51, determines the direction of movement of the detection engagement portion of the OCT scanning optical fiber assembly with respect to the said region to be detected, the said direction of movement comprising a rectilinear direction and/or a circumferential direction determined by the positioning point and a first boundary point and a second boundary point of the region to be detected.

Specifically, the controller is used to control the OCT scanning optical fiber assembly to move rectilinearly along the determined direction, in order to adjust the detection angle formed between the detection engagement portion and the region to be detected of the object to be examined to lie within a set range. The controller is used to control the OCT scanning optical fiber assembly to move rectilinearly along the determined direction and then to move circumferentially with respect to the centre of the object to be examined, so as to adjust the detection angle formed between the detection engagement portion and the region to be detected of the object to be examined to lie within a set range.

In one specific embodiment, the controller 40 is used to control the OCT scanning optical fiber assembly 20 to move rectilinearly along a determined direction (for example, along a direction parallel to the line segment sn of FIG. 6) (specifically, to move rectilinearly along the line segment sn), so as to adjust the detection angle a, formed between the detection engagement portion 21 and the region to be detected Q1 of the object to be examined 50, to lie within the set range, and to complete the detection process for the region to be detected Q1.

In another embodiment, the controller 40 is used to control the OCT scanning optical fiber assembly to move rectilinearly along a determined direction (for example, along a direction parallel to the line segment sz of FIG. 7) and then to move circumferentially with respect to the centre O of the object to be examined (specifically, along a curve zm), so as to adjust the detection angle a, formed between the detection engagement portion 21 and the region to be detected Q1 of the object to be examined, to lie within the set range.

Step S103: controlling, by means of the controller, the manipulator to grip and carry the OCT scanning optical fiber assembly so as to move along the determined rectilinear direction and/or circumferential direction, and to perform scanning detection on the object to be examined, in order to obtain a detection image of the region to be detected of the object to be examined.

Specifically, the OCT scanning optical fiber assembly is moved along the determined rectilinear direction and/or circumferential direction, and scanning detection is performed on the object to be examined, in order to obtain the detection image of the region to be detected of the object to be examined.

Next, a preset image recognition model is used to recognise the detection image obtained, in order to determine whether the detection image contains a target region, the target region being, for example, the macular region.

The foregoing has described preferred embodiments of the present invention; the said embodiments are not, however, intended to limit the present invention. Persons skilled in the art may, on the basis of the embodiments disclosed herein, make modifications and changes that do not depart from the scope and spirit of the present invention.

## Claims

1. An intraocular OCT optical fiber probe, comprising an OCT optical fiber, an optical fiber metal sleeve, a light-guide optical fiber, a rotating motor and a handle housing, wherein a head end of the OCT optical fiber and a head end of the light-guide optical fiber are fixedly packaged inside the optical fiber metal sleeve; the optical fiber metal sleeve is further connected to the rotating motor and is driven by the rotating motor to rotate, the optical fiber metal sleeve thereby driving the head end of the OCT optical fiber and the head end of the light-guide optical fiber to rotate; the rotating motor is electrically connected to a power line, the power line supplying electrical power to the rotating motor; at least a part of the optical fiber metal sleeve and the rotating motor are both arranged in the handle housing; a tail end of the light-guide optical fiber extends out of an end portion of the handle housing and is connected to an illumination light source, the light-guide optical fiber being used to provide illumination; and a tail end of the OCT optical fiber extends out of an end portion of the handle housing and is connected to an OCT examination device, the OCT optical fiber being used to carry out intraocular invasive OCT examination, **characterized in that** the OCT optical fiber comprises a single-mode optical fiber, a spring tube, a glass rod, a self-focusing lens and a reflector, the single-mode optical fiber being received in the spring tube; one end of the glass rod is cemented to the self-focusing lens at zero degrees, and the other end of the glass rod is obliquely cemented to the single-mode optical fiber; the single-mode optical fiber, the spring tube, the glass rod, the self-focusing lens and the reflector are sealed within the optical fiber metal sleeve, at least one reflective surface of the reflector forming a non-90° angle with the optical fiber; and an end portion of the optical fiber metal sleeve is sealed with UV glue.

2. The intraocular OCT optical fiber probe according to claim 1, wherein a gap between the head end of the OCT optical fiber and the head end of the light-guide optical fiber and an inner wall of the optical fiber metal sleeve is filled with an adhesive.

3. The intraocular OCT optical fiber probe according to claim 1, wherein the rotating motor drives the optical fiber metal sleeve to rotate, rather than directly rotating the optical fiber.

4. The intraocular OCT optical fiber probe according to claim 1, wherein the light-guide optical fiber is a multimode optical fiber.

5. The intraocular OCT optical fiber probe according to claim 1, wherein the light-guide optical fiber is a single-mode optical fiber.

6. The intraocular OCT optical fiber probe according to claim 1, wherein rotation of the optical fiber metal sleeve causes a scanning rotation angle of the intraocular OCT optical fiber to be from 1° to 360°.

7. The intraocular OCT optical fiber probe according to claim 1, wherein the optical fiber metal sleeve is a slotted stainless steel tube, a slot is provided in a side wall of the optical fiber metal sleeve, and a reflective surface of the reflector is directed toward an opening of the slot, in order to reduce the influence on imaging of stray light from the light source passing through a cylindrical inner tube of the optical fiber metal sleeve.

8. The intraocular OCT optical fiber probe according to claim 1, wherein the reflector is a cylindrical reflector.

9. An optical fiber scanning detection system for ophthalmology, comprising: a detection device for automatically detecting an object to be examined and a positioning point thereof; an OCT scanning optical fiber assembly, one end portion of which is connected to a lens structure, the OCT scanning optical fiber assembly being used to scan the position of the object to be examined and the position of the positioning point; a manipulator, used to grip and carry the OCT scanning optical fiber assembly so as to scan the object to be examined, and further to set down the OCT scanning optical fiber assembly at the positioning point or an indicated point of the object to be examined; and a controller, electrically connected to the detection device and the manipulator, the controller being used to control the detection device to perform automatic detection of the object to be examined and the positioning point thereof, the controller being used to control the manipulator to set down the OCT scanning optical fiber assembly at the positioning point or indicated point of the object to be examined, **characterized in that** the OCT scanning optical fiber assembly forms an implant-type detection structure with the object to be examined.

10. The optical fiber scanning detection system according to claim 9, wherein the lens structure includes a first lens, the optical axis direction of the first lens making a specified angle with the cross section of the OCT scanning optical fiber assembly, the specified angle being in the range of 0° to 90°.

11. The optical fiber scanning detection system according to claim 10, wherein the said one end portion of the OCT scanning optical fiber assembly is connected to the first lens, so that the specified angle formed between the optical axis direction of the first lens and the cross section of the OCT scanning optical fiber assembly is from 20° to 90°.

12. The optical fiber scanning detection system according to claim 9, wherein the OCT scanning optical fiber assembly includes a detection engagement portion; and the controller, on the basis of a region to be detected of the object to be examined, controls the manipulator to set down the detection engagement portion at the positioning point or indicated point of the object to be examined.

13. The optical fiber scanning detection system according to claim 12, wherein the detection engagement portion of the OCT scanning optical fiber assembly and the positioning point or indicated point of the object to be examined form an implant-type detection structure, the implant-type detection structure including a detection angle formed between the detection engagement portion and a region to be detected of the object to be examined.

14. The optical fiber scanning detection system according to claim 13, wherein the controller is further used to determine a direction of movement of the detection engagement portion of the OCT scanning optical fiber assembly on the basis of the automatically detected object to be examined and the positioning point thereof, and the region to be detected of the object to be examined.

15. The optical fiber scanning detection system according to claim 14, wherein the controller is used to control the OCT scanning optical fiber assembly to move rectilinearly along the determined direction so as to adjust the detection angle formed between the detection engagement portion and the region to be detected of the object to be examined to lie within a set range; or the controller is used to control the OCT scanning optical fiber assembly to move rectilinearly along the determined direction and then to move circumferentially with respect to a centre of the object to be examined, so as to adjust the detection angle formed between the detection engagement portion and the region to be detected of the object to be examined to lie within a set range.

16. The optical fiber scanning detection system according to claim 9, wherein the object to be examined is a sphere; and the optical fiber scanning system further comprises an illumination optical fiber, the illumination optical fiber being used to convey light from a visible light source for illuminating the region to be detected of the object to be examined.

17. An optical fiber scanning imaging method for ophthalmology, performed by using the optical fiber scanning detection system according to any one of claims 9 to 16, **characterized in that** the optical fiber scanning imaging method comprises: automatically detecting, by means of a detection device, an object to be examined and a positioning point thereof; determining, on the basis of a region to be detected and the determined positioning point, a direction of movement of a detection engagement portion of the OCT scanning optical fiber assembly with respect to the said region to be detected, the said direction of movement comprising a rectilinear direction and/or a circumferential direction determined by the positioning point and a first boundary point and a second boundary point of the region to be detected; and controlling, by means of a controller, the manipulator to grip and carry the OCT scanning optical fiber assembly to move along the determined rectilinear direction and/or circumferential direction, and to perform scanning detection on the object to be examined, in order to obtain a detection image of the region to be detected of the object to be examined.

18. The optical fiber scanning imaging method according to claim 17, further comprising: controlling, by means of the controller, the manipulator to set down the OCT scanning optical fiber assembly at the positioning point or indicated point of the object to be examined, so that the OCT scanning optical fiber assembly forms an implant-type detection structure with the object to be examined.
